# EUROPEAN PATENT APPLICATION

(11) **EP 4 703 008 A1**
(43) Date of publication of application: **04.03.2026**
(21) Application number: 24198058.0
(22) Date of filing: 03.09.2024
(51) Int. Cl.: A61N 5/06

(54) **A WEARABLE DEVICE AND A METHOD FOR STIMULATING SKIN AND FAT CELLS**

(71) Applicant: Messlender, Manfred, 9800 Spittal/Drau (AT); Kempf, Christopher, 9800 Spittal/Drau (AT)
(72) Inventor: Messlender, Manfred, 9800 Spittal/Drau (AT); Kempf, Christopher, 9800 Spittal/Drau (AT)
(74) Representative: Flügel Preissner Schober Seidel

(57) **Abstract**

A wearable device (100) is configured to be used by a person while exercising or relaxing, the wearable device (100) having an inner surface (I) and an outer surface (O), wherein the inner surface (I) is facing the person when wearing the wearable device, comprising: a lighting device (10) positioned on the inner surface (I), the lighting device (10) comprising a plurality of lighting units (14); wherein the lighting units (14) are configured to emit light for stimulating skin and fat cells; and a control unit (20) for controlling the lighting units (14); characterised in that each lighting unit (14) is allocated to at least one of a first section (S1) or a second section (S2); and wherein the light emission of the light unit (14) allocated to the first section (S1) is controlled by the control unit (20) to be different to the light emission of the light unit (14) allocated to the second section (S2).

## Description

The invention is directed at a wearable device and a method for stimulating skin and fat cells.

Contemporary therapeutic systems that engage in cellular stimulation, particularly targeting dermal and adipose tissues, utilize a variety of technologies that include light-based therapy. These systems aim to administer specific wavelengths to the tissues for therapeutic purposes. For example, low-level laser therapy (LLLT) is commonly used for non-invasive body contouring interventions. This therapy applies a light having a wavelength of 635nm to 680nm as disclosed by Caruso-Da-vis MK, et. al. in "Efficacy of low-level laser therapy for body contouring and spot fat reduction". Obes Surg. 2011 Jun;21 (6):722-9. doi: 10.1007/s11695-010-0126-y.

The most beneficial effects are achieved by using light therapy while exercising. The light therapy stimulates the cells in the area exposed to the light and consequently activates the fat burning in this area compared to untreated areas. In the area of the treated cells so called deep heat is generated and blood circulation is increased, thereby supporting lipolysis. For example, AT 524632 B1 discloses a belly belt for assisting lipolysis. The belly belt comprises a plurality of LED diodes. All LED diodes emit light simultaneously and in a uniform manner for treatment of the underlying skin.

The most beneficial effect for shaping the contour in defined body areas is achieved by use of light treatment in combination with a tailored endurance training. The tailored endurance training is generally defined and supervised by a personal coach and comprises several regular instances over a defined period. It is therefore desirable to reduce the required endurance training period or to improve the effectiveness of the light treatment to achieve a positive effect for the clients exercising with the personal coach.

The object of the present invention is to provide a wearable device and a method allowing for a shorter and/or more effective treatment.

The object of the invention is solved by the wearable device according to claim 1 and a method according to claim 12.

Advantageous embodiments are subject of the dependent claims.

An aspect of the invention is directed to a wearable device configured to be used by a person while exercising or relaxing. The wearable device has an inner surface and an outer surface. The inner surface is facing the person when wearing the wearable device. The wearable device comprises a lighting device and a control unit. The lighting device is positioned on the inner surface and comprises a plurality of lighting units. The lighting units are configured to emit light for stimulating skin and fat cells. The control unit is configured to control the lighting units.

The invention is characterized in that each lighting unit is allocated to at least one of a first section or a second section and that the light emission of the light unit allocated to the first section is controlled by the control unit to be different to the light emission of the light unit allocated to the second section.

The advantageous effect of the wearable device is that it provides targeted and customizable stimulation of skin and fat cells. By allocating each lighting unit to a specific section, the device can control the light emission of each section independently. This allows for different light treatments to be applied simultaneously, providing a more tailored and effective therapy. The ability to stimulate specific areas of the body with different light intensities and wavelengths enhances the therapeutic capabilities of the device, resulting in improved skin and fat cell stimulation.

Light emission refers to the process of producing and emitting light energy. In the context of the patent application, light emission specifically refers to the emission of light from the lighting units. The lighting units are designed to emit light for stimulating skin and fat cells. The light has a wavelength. Light with different wavelength have a different penetration depth and can thus treat different areas of the skin. In general, it can be said that the penetration depth increases with an increase of the wavelength. However, to activate a defined area within the skin it is crucial to apply the correct wavelength. A light with a wavelength up to 500 nm has a maximum penetration depth of around 1 mm and reaches the area of the epidermis and dermis. Light having a wavelength between 500 nm and 600 nm, also commonly referred to as green or yellow light, reaches the area of the dermis close to the subcutis and the subcutis. Light wave having a wavelength between 600 nm and 700 nm is commonly referred to as red light and has a penetration depth reaching the subcutis. Fat cells are commonly located in the area of the dermis and subcutis.

In one example of the different controlling of the first section and the second section, light could be emitted from the first section with a wavelength different to a wavelength of light emitted from the second section. Thereby, different areas in the skin due to different penetration depths can be treated allowing for a more bespoke application of the treatment. Hereto, a first section could be controlled to emit light having a wavelength between 500 nm and 600 nm thus activating the dermis region, while a second section could be controlled to emit light having a wavelength between 600 nm and 700 nm thus activating the subcutis region in the skin. Then, after a defined period of time the first section then emits the light previously emitted by the second section and, vice versa, the second section emits the light previously emitted by the first section. This alternating treatment further enhances the stimulation of the skin. In another possible example of the invention, a different treatment can result of light being emitted from the first section while no light is emitted from the second section. After a defined period of time, it could optionally be reversed such that no light is emitted from the first section and only light is emitted from the second section.

The shape of the first section and the second section are not specified and can be of any design. It is however advantageous to have multiple first sections and multiple second section spread over the lighting units. Furthermore, it is advantageous to have the first sections and the second sections positioned next to each other in an alternating design. However, it is also advantageous to have the first section overlapping with the second section to allow for simultaneous treatment of the same are with different light waves having different defined wavelengths.

Preferably, each lighting unit is allocated to the first section or the second section or a third section, wherein the light emission of the lighting unit allocated to the third section is controlled to be different to the light emission of the lighting unit allocated to the first section and the light emission of the lighting unit allocated to the second section.

The third section can emit an additional light having a wavelength different from the wavelength of the light emitted by the first section and the third section. However, it could also be controlled to not emit light, while the first section or the second section emits light, comparable to the previously described embodiment of only two sections. Similarly, it is possible to have multiple first sections, multiple second sections, and multiple third sections alternately distributed across the lighting device. The alternation can be a repetitive sequence or a random sequence. In a further enhanced method, it is also possible to have a fourth section or more sections. The additional section allows for a more specific treatment with an even more enhanced training effect.

Preferably the first section and/or the second section and/or the third section each comprise at least two lighting units. The more lighting units a section contains, the higher the light intensity can be. In addition, a larger lighting unit can be produced, if more lighting units are comprised in each section.

Preferably the lighting units allocated to one section are positioned along a row. This allows for an alternating distribution of the sections along the width of the lighting device.

Preferably the row of lighting units allocated to the first section is parallel to the row of lighting units allocated to the second section. Even more preferably the row comprising the first section and the row comprising the second section are positioned in parallel to the row of lighting units allocated to the third section.

This specific arrangement of parallel alternating distributed rows allow to further enhance the training effect as fat and skin cells can be activated in alternating way such that through the alternation in nearby areas blood flow can even further be enhanced.

Preferably the wearable device comprises on the inner surface a transparent top cover designed to cover the lighting device. This top cover allows for easy cleaning of the wearable device. Furthermore, the lighting device is also protected. In particular, during sweating the lighting device can be protected from the sweat, thereby providing a more hygiene device. In addition, the top cover may be attached to the wearable device along the outer edge of the top cover. The top cover may include an opening for insertion of the lighting device. The opening can be closed with a zipper.

Preferably the base is designed to be used as a belt configured to be placed around a person's belly or as a mask configured to be placed on a face or as shorts. The problem areas for weight loss are located in the belly area. Therefore, a belly belt allows to target the body parts in this area. The same goes for the shorts treating the body parts near the legs. The face mask can be used for relaxation and further treatment of the eye area.

Preferably the lighting device comprises a lighting panel having the lighting units positioned thereon, and a top cover and/or a back cover covering the lighting panel. The lighting panel becomes more stable by use of a back cover. The back cover can be made of a stable material such as rubber with a thickness of 5 mm.

This keeps it in shape, but at the same time remains flexible and does not interfere with endurance training. The front cover has transparent sections to allow the light from the lighting units to shine through. However, the top cover further protects the lighting units to allow for intensive and safe use during exercise.

Preferably the lighting unit is configured to emit light having a wavelength between 280nm and 1400nm, preferably between 500nm and 600nm, most preferably having a wavelength of 569nm. The green light treats as outlined above the area in the dermis and subcutis. It was found that the range of 500 nm to 600 nm and specifically 569 nm shows the most beneficial effect of fat burning and most efficient results in a shorter period of time when combined with a personal endurance training.

Preferably the lighting unit is configured to emit a pulsed light wave, wherein preferably the pulsed light wave comprises a main wave including a plurality of sub waves. The sub waves may comprise different wavelengths. Thereby a more intense treatment can be enabled. Furthermore, it was shown that the device is more energy efficient. The use of pulsed light waves allows in particular for the use of a mask to have a variable intensity. Thereby it is possible to apply in a first mode an anti-inflammatory treatment, in a second mode an anti-headache treatment and in a third mode a combination of the first and second mode treatment. The modes mas also be selected by use of a controller as outlined in the following.

Preferably is the control unit configured to control the light emitted according to a first mode or a second mode or preferably a third mode. In a first mode, the focus of the light treatment can be directed to the fat cells. In a second mode, the focus of the light treatment can be directed to the skin cells. In a third mode, the focus of the light treatment can be directed at both, the fat cells and the skin cells. This allows for a more bespoke and variable treatment.

A further aspect of the invention is directed at a method for stimulating skin and fat cells using a wearable device having an inner surface facing a person while wearing the wearable device and an outer surface. The wearable device comprises a lighting device including lighting units configured to emit light for stimulating skin cells, wherein the lighting units are allocated to at least one of a first section or a second section. The device further comprises a control unit for controlling the lighting units. The method comprising the following steps: a) receiving the lighting device on the inner surface; b) controlling the lighting units to emit light from the first section differently from the second section for stimulating the skin and fat cells.

The method is in particular directed to the use of the wearable device. It is highlighted that the use of the wearable device during an exercise allows the targeted fat burning in defined body areas.

Preferably the method also includes the step of controlling the lighting units allocated to the first section and the second section to emit light simultaneously after or before previous step b). This alteration in different modes allow for a more enhanced use according to the individual needs of the person training with the wearable device.

Preferably is the emitted light a pulsed wave. Preferably the pulsed wave comprises plurality of sub waves, wherein each of the pulsed sub waves comprise a different wavelength. As outlined above this allows to treat different areas along the penetration depth into the skin.

In the following, a wearable device and a method for stimulating skin and fat cells as well as further features and advantages are explained in more detail with reference to embodiment examples, which are shown schematically in the figures. The figures show:
- FIG. 1: shows the inner surface of the wearable device;
- FIG. 2: shows the outer surface of the wearable device;
- FIG. 3: shows the lighting device according to a first embodiment;
- FIG. 4: shows the lighting device according to a second embodiment;
- FIG. 5: shows a section of the lighting device;
- FIG. 6: shows a pulsed wave;
- FIG. 7: shows the same lighting device at two different times;
- FIG. 8: shows an exploded perspective view of the lighting device;
- FIG. 9: shows the control unit;
- FIG. 10: shows a wearable device as a mask;
- FIG. 11: shows the inner surface of a wearable device as a shorts; and
- FIG. 12: shows the outer surface of a wearable device of Fig. 11.

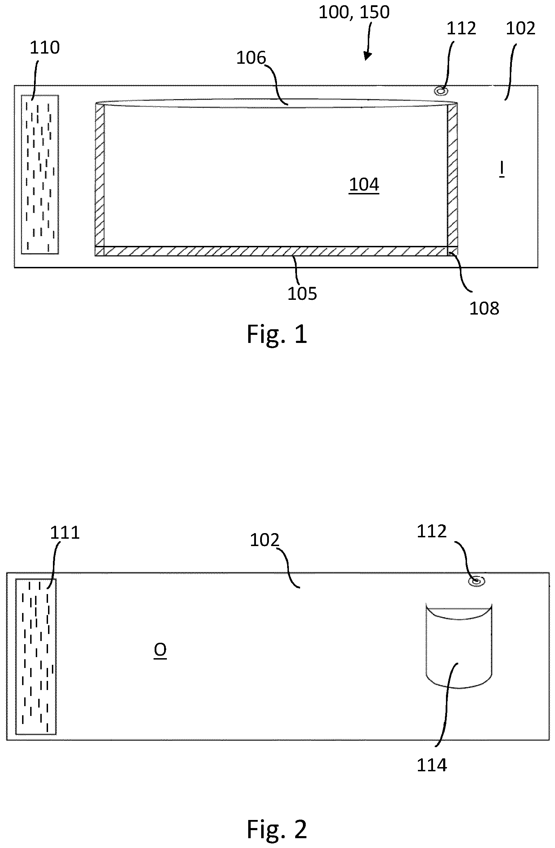

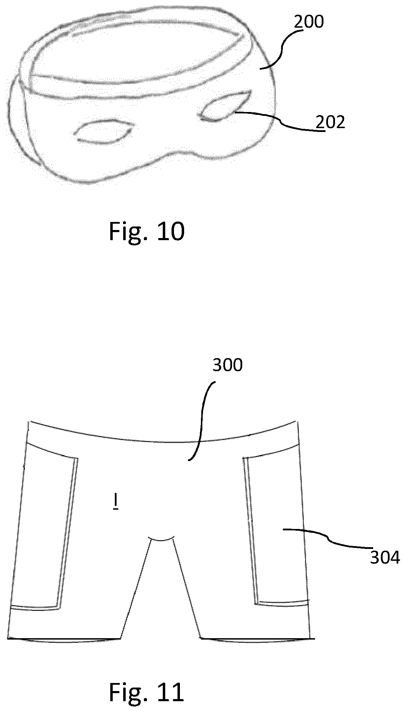

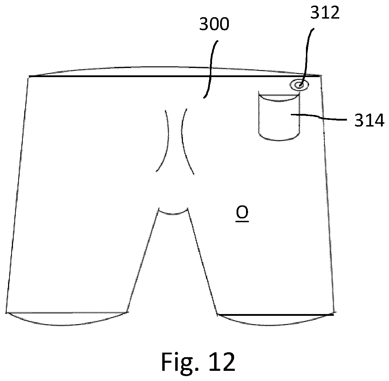

FIG. 1 and Fig. 2 illustrate one embodiment of a wearable device 100 as a belt 150 which can be placed around a belly or abdomen. Further examples of a wearable device are shown in Fig. 10 in the form of a mask 200 for a face comprising eye openings 202 and in Fig. 11 and Fig. 12 as shorts 300. The features described below with respect to the wearable device 100 as a belt 150 apply also to the other described embodiments of wearable devices. Reference signs of the mask 200 correspond to the reference sing of the belt 150. The same applies to the shorts 300 compared to the belt 150. For example, the base 102 of the belt 150 as a wearable device 100, the base 202 of the mask 200 and the base 302 of the shorts 300 refer to a feature that fulfils the same functional task of supporting a lighting device 10 in place with respect to the body part of a person.

The wearable device 100 comprises a base 102 and has an inner surface I and an outer surface O. The base 102 provides structural support for the wearable device 100 and may be configured to conform to a portion of the human body, in the present case an abdomen. A top cover 104 is attached to the inner surface I of the base 102. The top cover 104 is made of a translucent material that allows light waves to pass through. The top cover 104 comprises an edge 105. The edge 105 is fixed to the base 102. The Edge 105 can be sewn or glued to the base 102. In this case, it is sewn so as to form a seam 108. The top cover 104 includes an opening 106 for receiving the lighting device 10. The opening 106 can be closed with a zipper. The top cover 104 forms a pocket for the lighting device 10, thereby protecting and positioning the lighting device 10. In addition, the use of the top cover 104 forms a smooth inner surface which increases the comfort of wearing the wearable device for the user.

The wearable device further comprises an orifice 112 to allow a cable 25 or a cable 35 to be connected to an energy storage unit 30 placed on the outer surface O in a pocket 114. The orifice 112 can also be covered by the top cover 104.

To hold the wearable device as shown in Fig. 1 and Fig. 2 around a belly, the wearable device comprises on the inner surface and on the outer surface a Velcro. The Velcro is a hook-and-loop fastener 110. This allows to easily position the wearable device in its embodiment as a belly belt 150 around the abdomen.

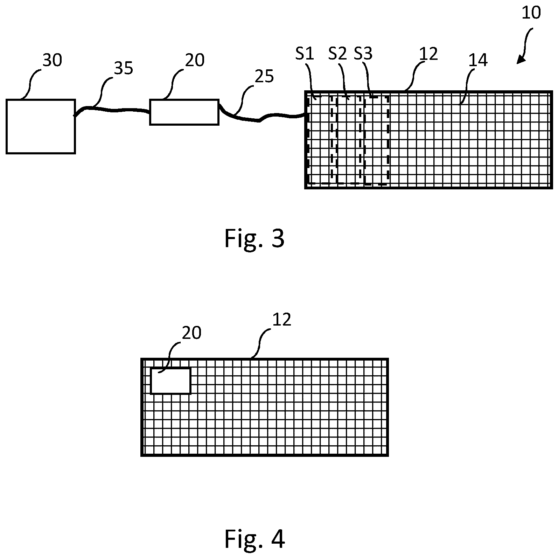

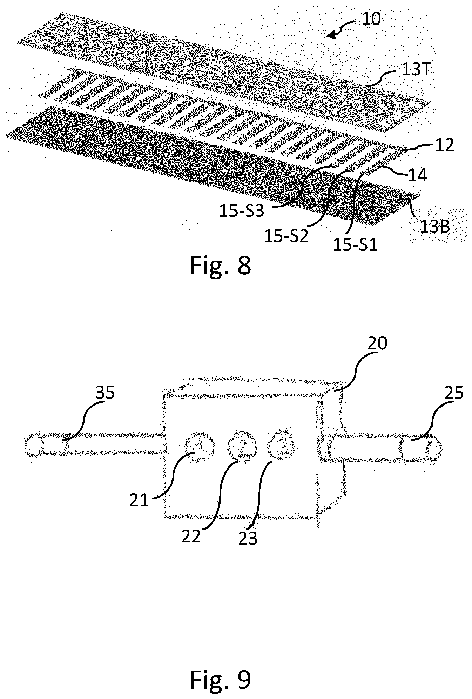

The lighting device 10 is designed to emit light for stimulating skin and fat cells and is shown in more detail in Fig. 3. The lighting device 10 comprises a lighting panel 12, a control unit 20 and an energy storage unit 30. The energy storage unit 30 supplies electricity via a cable 35 to the control unit 30 and via a cable 25 to the lighting panel 12 and the lighting units 14. In an alternative embodiment is it also possible to have the control unit 20 integrated in the lighting panel 12 as shown in Fig. 4. The control unit 20 is shown in more detail in Fig. 9. As shown in Fig. 9 the control unit 20 may comprise a first mode button 21, a second mode button 22 and a third mode button 23. Each mode button activates a different treatment mode. This allows a more tailored and individual and specific application of the wavelength, the intensity, or the duration of the application of light emission.

The lighting panel 12 comprises lighting units 14. The lighting units 14 emit light having a wavelength. The light can be in a visible range but also in a non-visible range such as infra-red light. The lighting units 14 arranged on the lighting panel 12 are allocated to a first section S1 or a second section S3 or preferably as shown in Fig. 3 to a third section S3. Each section S1, S2, S3, and in particular each lighting unit 14 allocated within the corresponding section S1, S2, S3, can be controlled separately by the control unit 20.

An exploded view of the lighting device 10 including the lighting panel 12 is shown in more detail in Fig. 8. As shown therein the lighting panel 12 is covered by a top cover 13T and a back cover 13B. The top cover 13T comprises transparent sections to allow light emitted from the lighting units 14 to shine therethrough.

The lighting units 14 of one section S1, S2, S3 are arranged on the lighting panel 12 along a single row 15-S1, 15-S2, 15-S3. The rows 15-S1, 15-S2, 15-S3 are arranged in parallel to each other. The third section S3 shown in this embodiment is an optional feature.

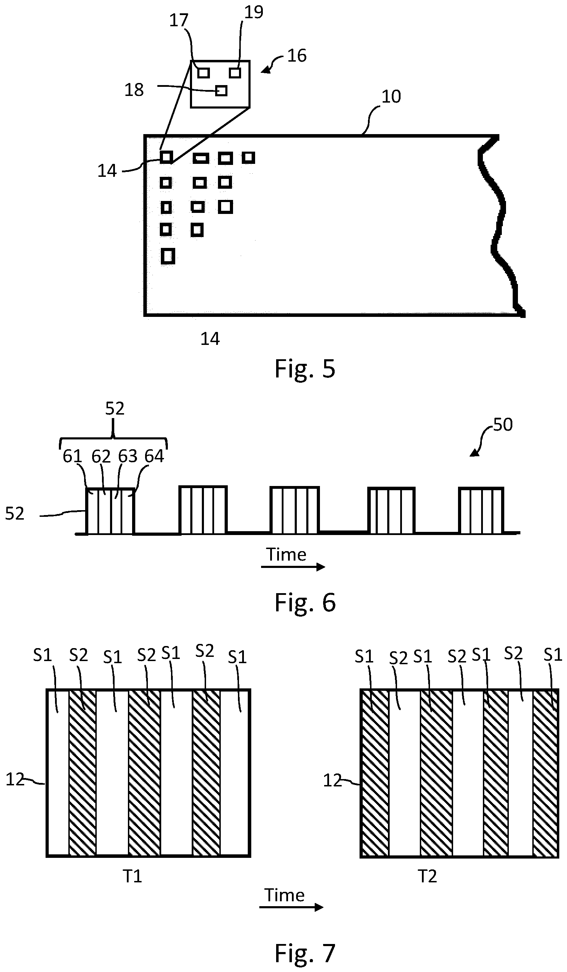

The lighting unit 14 is shown in more detail in Fig. 5. The lighting unit 14 as shown in this embodiment is a LED-unit 16 having three LEDs. A LED is a light emitting diode. The embodiment shown with three LEDs 17, 18, 19 is optional. An embodiment having only one or two LEDs 17, 18, 19 is also possible. The option with three LEDs 17, 18, 19 have the advantage that all these LEDs can be controlled to emit light with a different wavelength. For example, the LED 17 can be configured to emit light with a wavelength between 500 nm and 600 nm, the LED 18 can be configured to emit light with a wavelength between 600 nm and 700 nm and the LED 19 can be configured to emit light with a wavelength of more than 700 nm. Having more than one LED unit activated simultaneously allows to have overlapping sections.

The emitted light of the lighting unit 14 can be a pulsed light wave 50 as shown in Fig. 6. The pulsed light wave 50 comprises a main wave 52 which comprises a multiple of sub waves 61 to 64. The sub waves 61 to 64 each may comprise a different wavelength. Hence, different areas in the skin can be treated with a single main wave 52. Further, as no light is emitted between two main waves 52 no energy is consumed hence allowing for a longer working time of the wearable device 100.

Fig. 7 shows the panel in use with a first section S1 and a second section S2 at two different steps in time. The shaded area represents an activated area. At time T1 only the second sections S2 are activated. At time T2 the first sections S1 are activated. Each period of time T1, T2 lasts for a few seconds. This allows for an alternating treatment.

Overall, the wearable device as outlined above allows for an efficient treatment of the skin cells. This treatment of allowing to treat different areas enable an enhanced training effect such that the fat can be burned in the treated area more specific to the client's needs. Hence, the coached endurance training can be targeted specifically to the individual needs.

### Reference signs

- 10: Lighting device
- 12: Lighting panel
- 13T: Top cover
- 13B: Back cover
- 14: lighting unit
- 15-S1: row within first section
- 15-S2: row within second section
- 15-S3: row within third section
- 16: LED-unit
- 17: Red-LED
- 18: Green-LED
- 19: Non-visible-LED

- S1: First section
- S2: Second section
- S3: Third section

- 20: Control unit
- 21: First mode button
- 22: Second mode button
- 23: Third mode button
- 25: Cable

- 30: Energy storage unit
- 35: Cable

- 50: Pulsed light wave
- 52: Main Wave
- 61: First sub wave
- 62: Second sub wave
- 63: Third sub wave
- 64: Fourth sub wave

- 100: Wearable device
- 102: Base
- 104: Top cover
- 105: Edge
- 106: Opening
- 108: Seam
- 110: Velcro
- 111: Velcro
- 112: Orifice
- 114: Pocket
- I: inner surface
- O: Outer surface

- 150: Belt

- 200: Face mask
- 202: Eye opening

- 300: Shorts
- 304: Top Cover
- 312: Orifice
- 314: Pocket

## Claims

1. A wearable device (100) configured to be used by a person while exercising or relaxing, the wearable device (100) having an inner surface (I) and an outer surface (O), wherein the inner surface (I) is facing the person when wearing the wearable device, comprising:
a lighting device (10) positioned on the inner surface (I), the lighting device (10) comprising a plurality of lighting units (14);
wherein the lighting units (14) are configured to emit light for stimulating skin and fat cells; and
a control unit (20) for controlling the lighting units (14);
**characterised in that** each lighting unit (14) is allocated to at least one of a first section (S1) or a second section (S2); and
wherein the light emission of the light unit (14) allocated to the first section (S1) is controlled by the control unit (20) to be different to the light emission of the light unit (14) allocated to the second section (S2).

2. The wearable device (100) according to claim 1, wherein each lighting unit (14) is allocated to the first section (S1) or the second section (S2) or a third section (S3), wherein the light emission of the lighting unit (14) allocated to the third section (S3) is controlled to be different to the light emission of the lighting unit (14) allocated to the first section (S1) and the light emission of the lighting unit (14) allocated to the second section (S2).

3. The wearable device (100) according to claims 1 or 2, wherein the first section (S1) and/or the second section (S2) and/or the third section (S3) each comprise at least two lighting units (14).

4. The wearable device (100) according to claim 3, wherein the lighting units (14) allocated to one section (S1, S2, S3) are positioned along a row (15-S1, 15-S2, 15-S3).

5. The wearable device (100) according to claim 3, wherein the row (15-S1) of lighting units (14) allocated to the first section (S1) is parallel to the row (15-S2) of lighting units (14) allocated to the second section (S2), and preferably also parallel to the row (15-S3) of lighting units (14) allocated to the third section (S3).

6. The wearable device (100) according to any one of claims 1 to 5, wherein the wearable device (100) comprises on the inner surface (I) a transparent top cover (104) designed to cover the lighting device (10).

7. The wearable device (100) according to any one of claims 1 to 6, wherein the base (102) is designed to be used as a belt (150) configured to be placed around a person's belly or as a mask (200) configured to be placed on a face or as shorts (300).

8. The wearable device (100) according to any one of claims 1 to 7, wherein the lighting device (10) comprises a lighting panel (12) having the lighting units (14) positioned thereon, and a top cover (13T) and/or a back cover (13B) covering the lighting panel (12).

9. The wearable device (100) according to any one of claims 1 to 8, wherein the lighting unit (14) is configured to emit light having a wavelength between 280nm and 1400nm, preferably between 500nm and 600nm, most preferably having a wavelength of 569nm.

10. The wearable device (100) according to any one of claims 1 to 9, wherein the lighting unit (14) is configured to emit a pulsed light wave (50), wherein preferably the pulsed light wave (50) comprises a main wave (52) including a plurality of sub waves (54).

11. The wearable device (100) according to any one of claims 1 to 10, wherein the control unit (20) is configured to control the light emitted according to a first mode or a second mode.

12. A method for stimulating skin and fat cells using a wearable device (100) having an inner surface (I) facing a person while wearing the wearable device (100) and an outer surface (O), wherein the wearable device (100) comprises a lighting device (10) including lighting units (14) configured to emit light for stimulating skin cells, wherein the lighting units (14) are allocated to at least one of a first section (S1) or a second section (S2); and a control unit (20) for controlling the lighting units (14); the method comprising the following steps:
a) receiving the lighting device (10) on the inner surface (I);
b) controlling the lighting units (14) to emit light from the first section (S1) differently from the second section (S2) for stimulating the skin and fat cells.

13. The method according to claim 12, **characterised by** controlling the lighting units (14) allocated to the first section (S1) and the second section (S2) to emit light simultaneously after or before step b).

14. The method according to claim 12 or 13, wherein the emitted light is a pulsed wave (50).

15. The method according to claim 14, wherein the pulsed wave (50) comprises plurality of sub waves (54), wherein each of the pulsed sub waves (54) comprise a different wavelength.
